# EUROPÄISCHE PATENTSCHRIFT

(11) **EP 0 636 376 B1**
(45) Veröffentlichungstag und Bekanntmachung des Hinweises auf die Patenterteilung: **13.01.1999**
(21) Anmeldenummer: 94110927.4
(22) Anmeldetag: 13.07.1994
(51) Int. Cl.: A61L 2/22, A01N 25/30, C11D 3/48, C09K 3/30

(54) **Verfahren zum Reinigen und/oder Desinfizieren und Pflegen eines hohlen oder rohrförmigen medizinischen Behandlungsinstruments und Mittel zur Ausführung des Verfahrens**
Process for cleaning and/or disinfecting and protecting an especially hollow or tubular instrument for medical treatment and agent therefor
Procédé de purification et/ou désinfection et entretien d'un instrument à traitement médical particulièrement creux ou tubulaire et agent pour sa mise en oeuvre

(30) Priorität: 26.07.1993 DE 4325046
(43) Veröffentlichungstag der Anmeldung: 01.02.1995
(73) Patentinhaber: KALTENBACH & VOIGT GmbH & Co., 88400 Biberach/Riss (DE)
(72) Erfinder: Eibofner, Eugen, D-88400 Biberach (DE); Kuhn, Bernhard, D-88433 Schemmerhofen (DE)
(74) Vertreter: Schmidt-Evers, Jürgen, Dipl.-Ing.

(56) Entgegenhaltungen:
- DE-A- 2 916 552
- ROEMPP CHEMIE LEXIKON, Georg Thieme Verlag, 9. Ausgabe, 1992, Stuttgart (DE); "Polituren", Seite 351, "Putzmittel", Seiten 3687-3688
- ROTE LISTE; "Sterillium Virugard", no. 33034

## Beschreibung

Die Erfindung bezieht sich auf ein Verfahren nach dem Oberbegriff des Anspruchs 1 sowie ein Mittel zur Ausführung des Verfahrens.

Zur Behandlung des menschlichen oder tierischen Körpers werden verschiedenartige Behandlungsinstrumente benutzt, durch die Körperflüssigkeiten entfernt oder Behandlungsmedien zugeführt werden, wie es z.B. bei einem ärztlichen oder zahnärztlichen Handstück zur Behandlung des Körpers der Fall ist. Bei einem solchen Handstück Kann es sich um ein Behandlungsinstrument zur mechanischen oder auch anderweitigen Behandlung des Körpers handeln, wie z.B. ein Bohr- oder Fräshandstück, ein Spritzhandstück oder auch eine Sonde.

In allen vorgenannten Fällen ist nach der Behandlung eine Reinigung und/oder Desinfektion des Handstücks erforderlich, um eine Verschmutzung oder Kontaminierung des Handstücks aufzuheben.

Bei hohlen Behandlungsinstrumenten ist eine Reinigung und/oder Desinfektion und Pflege schwierig, weil die Reinigung, Desinfektion und Pflege auch in kleinen Spalten und Ritzen des Hohlraums des Behandlungsinstruments erfolgen muß, um zum einen eine Verschmutzung oder Kontaminierung des nächsten zu behandelnden Körpers zu vermeiden und zum anderen eine vollständige innere Pflege des Behandlungsinstruments zu gewährleisten, was für dessen Lebensdauer und Funktionstüchtigkeit von wesentlicher Bedeutung ist.

In der DE-A-40 24 171 A1 ist ein Verfahren zum Pflegen von ärztlichen und zahnärzlichen Instrumenten beschrieben, bei dem die Instrumente durch Ausblasen gereinigt und durch Auskochen in einem Wasserbad desinfiziert werden. Anschließend erfolgt eine Innentrocknung der Instrumente durch Ausblasen mit Heißluft. Durch eine solche Behandlung wird ein Schmiermittel zwischen mechanisch zusammenwirkenden Teilen im Instrument beseitigt. Um den Lauf der mechanisch zusammenwirkenden Teile des Instruments zu gewährleisten, wird nach der Innentrocknung Öl in das Instrument durch Heißluft eingeblasen. Durch ein nachfolgendes Ausblasen des Instruments mit Heißluft wird die Schmierung intensiviert und außerdem überschüssiges Öl aus dem Hohlraum des Instruments entfernt. Eventuell zur Außenseite des Instruments gelangtes Öl kann danach durch eine Reinigung mittels Tensidzusatz im Sinne einer Außenreinigung beseitigt werden.

Ein vergleichbares Verfahren zur Pflege ärztlicher und/oder zahnärztlicher Instrumente ist in der DE 41 25 223 A1 beschrieben.

Bei verschiedenen Behandlungsinstrumenten sind mehrere Hohlräume vorhanden, die unterschiedlichen Zwecken dienen. Z.B. bei einem ärztlichen oder zahnärztlichen Behandlungsinstrument gibt es Hohlräume zur Aufnahme von Antriebsteilen, wie eine Antriebswelle oder ggf. auch ein Getriebe, und für die Durchführung von Medien, insbesondere Behandlungsmedien wie Luft, Wasser oder Spray.

In der älteren Patentanmeldung P 42 35 699.7 der Anmelderin ist eine Vorrichtung und ein Verfahren zum Reinigen und/oder Desinfizieren und/oder Pflegen von ärztlichen oder zahnärztlichen Handstücken beschrieben. Bei dieser Vorrichtung erfolgt die Behandlung des bzw. der Behandlungsinstrumente im Wasserbad eines Spülbehälters, dem das Spülwasser durch eine Zuführungs- und Abführungsleitung zugeführt, auf einem bestimmten Niveau gehalten und abgeführt werden kann. Im Spülbehälter sind Halterungen zum Halten der Behandlungsinstrumente in aufrechter Position angeordnet. Dabei sind jeder Halterung zwei Zuführungsleitungen zugeordnet, die beim Einsetzen des Behandlungsinstruments in die zugehörige Halterung, z.B. durch Einstecken oder oder Aufstecken in oder auf eine Fassung, mit den verschiedenen vorbeschriebenen Hohlräumen im Behandlungsinstrument verbunden werden. Beim Reinigen und/oder Desinfizieren wird das Behandlungsinstrument zunächst in einem heißen Wasserbad außen und innen gereinigt und ggf. auch desinfiziert, was durch die Hitze und/oder ein dem Wasser zugeführtes Desinfektionsmittel erfolgt. Die Innenreinigung wird durch ein Ausblasen des Behandlungsinstruments mit vorzugsweise heißer Luft forciert, wobei auch ein Austrocknen der Hohlräume stattfinden kann. Bei dieser Vorrichtung und diesem Verfahren wird das Pflegemittel nur dem Hohlraum des Behandlungsinstruments zugeführt, der mechanisch miteinander zusammenwirkende Teile enthält, die gepflegt und geschmiert werden sollen. Hierzu ist durch die zugehörige Zuführungsleitung ein Pflegemittel, vorzugsweise Öl, einblasbar, und zwar insbesondere durch Heißluft. Ein sich wieder Füllen wenigstens dieses Hohlraums mit Spülwasser kann durch die Zuführung von Druckluft durch die zugehörige Zuführungsleitung erfolgen, die im Hohlraum einen Druck aufbaut und dadurch das selbsttätige Eindringen von Spülwasser in diesen Hohlraum verhindert.

Bei allen vorbeschriebenen Verfahren zum Pflegen besteht eine Schwierigkeit darin, daß der oder die zu reinigenden und/oder zu desinfizierenden und zu pflegenden Hohlräume im Behandlungsinstrument von schwieriger Formgebung sein können und auch enge Spalten, Kanäle und Schlitze aufweisen können, wie es insbesondere dann der Fall ist, wenn in dem Hohlraum Antriebs- oder Lagerteile angeordnet und gelagert sind, wie z.B. eine Antriebswelle mit Wälzlagern und ggf. auch ein Getriebe mit Zahnrädern. In einem solchen Fall ist es schwierig und zeitraubend, nach dem Reinigen und/oder Desinfizieren mit der betreffenden Flüssigkeit diese aus dem Hohlraum wieder zu entfernen, da sich kleine Flüssigkeitsansammlungen, Benetzungen oder Tröpfchen insbesondere in Ecken und Spalten, Kanälen oder zwischen miteinander in Verbindung stehenden Antriebs- und/oder Lagerelementen ansammeln können. Wenn anschließend das Pflegemittel zugeführt wird, kann es nicht an die mit der Reinigungs- und/oder Desinfektionsflüssigkeit benetzten oder besetzten Stellen gelangen. Die Folge davon ist eine unzureichende Pflege, die zu einer frühzeitigen Korrosion und Verschleiß am Behandlungsinstrument führt.

Aus der DE-A-2 916 552 ist ein aus einem Behälter versprühbares Pflegemittel für ärztliche Instrumente bekannt, welches aus einer Mischung aus Öl und Treibmittel sowie gegebenenfalls Reinigungsmittel besteht. Die Mischung enthält 5 bis 15 Gew. % einer alkoholaldehydischen Wirkstoffkombination, die als Sterilisationshilfe dient.

Allgemein ist bereits ein flüssiges Reinigungs- und/oder Desinfektionsmittel bekannt, welches ein Mittel enthält, das eine Vermischung des Reinigungs- und/oder Desinfektionsmittels mit einem Pflegemittel gewährleistet (siehe z.B. RÖMPP CHEMIE LEXIKON, 9. Auflage, Paragraphen "Putzmittel" oder "Polituren").

Der Erfindung liegt die Aufgabe zugrunde, ein Verfahren der eingangs beschriebenen Art so weiterzubilden, daß eine sichere Pflege erreicht wird, und ein Mittel zur Ausführung dieses Verfahren anzugeben.

Diese Aufgabe wird durch die Merkmale des Anspruchs 1 gelöst. Des weiteren wird ein Mittel zur Durchführung des erfindungsgemäßen Verfahrens vorgeschlagen, wobei das Mittel die Merkmale des Anspruches 8 aufweist.

Beim erfindungsgemäßen Verfahren wird eine Reinigungs- und/oder Desinfektionsflüssigkeit und/oder ein Pflegemittel verwendet, die bzw. das ein Mittel enthält, das eine Vermischung der Reinigungs- und/oder Desinfektionsflüssigkeit mit dem Pflegemittel gewährleistet. Bei diesem Mittel kann es sich z.B. um ein Lösungsmittel oder einen Emulgator handeln, der die Oberflächenspannung herabsetzt und dadurch die angestrebte Vermischung herbeiführt. Aufgrund der Vermischung erfolgt die Pflege auch an den Stellen, an denen die Reinigungs- und/oder Desinfektionsflüssigkeit die Wandung des Behandlungsinstruments benetzt oder Tröpfchen davon anhaften.

Der durch die Erfindung erzielbare Vorteil beruht nicht nur auf der vorbeschriebenen Gewährleistung der Pflege, sondern auch darauf, daß der Aufwand zum Entfernen der Reinigungs- und/oder Desinfektionsflüssigkeit vom Behandlungsinstrument verringert werden kann und somit die Pflege schneller und dabei sicher durchgeführt werden kann. Es ist z.B. möglich, auf eine Trocknung nach dem Reinigen und/oder Desinfizieren zu verzichten, da auch benetzte oder durch Tröpfchen besetzte Flächen bei dem erfindungsgemäßen Verfahren sicher gepflegt werden.

Die Erfindung ist nicht auf hohle oder rohrförmige Behandlungsinstrumente beschränkt. Das der Erfindung zugrundeliegende Problem stellt sich auch an Außenflächen des Behandlungsinstruments und zwar insbesondere dann, wenn es außenseitige Schlitze oder Engstellen aufweist, an denen die Reinigungs- und/oder Desinfektionsflüssigkeit leicht haften bleibt.

Als Pflegemittel eignet sich ein auf dem vorliegenden Fachgebiet übliches Schmier-Öl bzw. Dental-Öl sehr gut. Bei dem erfindungsgemäßen Pflegemittel kann es sich jedoch auch um einen Pflege-Spray handeln.

Es hat sich bei Versuchen gezeigt, daß bereits dann eine akzeptable Vermischung erreicht wird, wenn das Mittel in einer Mischung von etwa 0,5% bis 15%, insbesondere etwa 1% bis 10%, vorzugsweise etwa 4% oder 5% bis 8% beigefügt ist.

Es hat sich im weiteren gezeigt, daß sich ein ethoxiliertes Olein als Emulgator gut eignet.

Bei dem erfindungsgemäßen Mittel bzw. Emulgator kann es sich um eine Kombination aus Fettaminpolyglykol und ethoxiliertem Olein oder um einen Polyalkrylenglykolether ethoxyliert handeln, inbesondere in den vorgenannten Gewichts- % - Bereichen. Dabei hat es sich im weiteren gezeigt, daß im ersten Fall sich ein Gewichts - % - Anteil von etwa 4% und im zweiten Fall von etwa 8% gut eignet. Außerdem ist bei diesen Emulgatoren die biologische Abbaubarkeit gewährleistet.

Das erfindungsgemäße Verfahren eignet sich sowohl für eine manuelle als auch maschinelle Reinigung und/oder Desinfektion und Pflege des Behandlungsinstruments, z.B. dadurch daß die Innenreinigung und/oder Innendesinfektion durch eine Durchspülung oder -blasung der Reinigungs- und/oder Desinfektionsflüssigkeit erfolgen kann, oder dadurch, daß die Reinigung und/oder Desinfektion in einem Reinigungs-und/oder Desinfektionsbad erfolgt, wie eingangs beschrieben. Das erfindungsgemäße Verfahren ist insbesondere in Kombination mit den eingangs gewürdigten Verfahren vorteilhaft.

Das erfindungsgemäße Verfahren läßt sich sowohl dann vorteilhaft ausführen, wenn das erfindungsgemäße Mittel im Pflegemittel enthalten ist, als auch dann, wenn es im Reinigungs- und/oder Desinfektionsmittel enthalten ist, weil das betreffende Gemisch in einem Behälter fertig angemischt zur Verfügung gestellt werden kann. Da die Menge des erforderlichen Pflegemittels gering ist, empfiehlt es sich, das Mittel in das Pflegemittel einzumischen. Wenn es sich bei der Reinigungs- und/oder Desinfektionsflüssigkeit um Wasser handelt, ist es vorteilhaft und einfacher, es aus einer üblichen Wasserversorgung zu entnehmen.

## Patentansprüche

1. Verfahren zum Reinigen und/oder Desinfizieren und Pflegen eines insbesondere hohlen oder rohrförmigen medizinischen Behandlungsinstruments, insbesondere eines ärztlichen oder zahnärztlichen Behandlungsinstruments wie eines Handstücks, bei dem das Behandlungsinstrument mit einem vorzugsweise flüssigen Reinigungs- und/oder Desinfektionsmittel gereinigt und/oder desinfiziert und dann mit einem Pflegemittel gepflegt wird,
**dadurch gekennzeichnet**,
daß ein Reinigungsmittel und/oder ein Desinfektionsmittel und/oder ein Pflegemittel verwendet wird, in dem bzw. in denen ein Mittel enthalten ist, das eine Vermischung des Reinigungs- und/oder Desinfektionsmittels mit dem Pflegemittel gewährleistet.

2. Verfahren nach Anspruch 1,
**dadurch gekennzeichnet**,
daß als Mittel im Reinigungs- und/oder Pflegemittel ein Lösungsmittel oder Emulgator verwendet wird.

3. Verfahren nach Anspruch 1 oder 2,
**dadurch gekennzeichnet**,
daß als Pflegemittel ein Öl oder ein Spray verwendet wird.

4. Verfahren nach einem der Ansprüche 1 bis 3,
**dadurch gekennzeichnet**,
daß ein Mischungsverhältnis für das Mittel von etwa 1% bis 10%, insbesondere etwa 5%, in der Flüssigkeit und/oder im Pflegemittel benutzt wird.

5. Verfahren nach einem der vorhergehenden Ansprüche,
**dadurch gekennzeichnet**,
daß als Mittel ein ethoxiliertes Olein verwendet wird.

6. Verfahren nach einem der vorhergehenden Ansprüche,
**dadurch gekennzeichnet**,
daß das Pfegemittel manuell oder in einer gesteuerten Vorrichtung zum Reinigen und/oder Desinfizieren und Pflegen des Behandlungsinstruments eingeführt oder eingeblasen wird.

7. Verfahren nach einem der vorhergehenden Ansprüche,
**dadurch gekennzeichnet**,
daß es in einem Spülbehälter, vorzugsweise im mit der Reinigungs- und/oder Desinfektionsflüssigkeit gefüllten Spülbehälter durchgeführt wird.

8. Pflegemittel oder flüssiges Reinigungs- und/oder Desinfektionsmitttel zur Ausführung des Verfahrens nach einem der vorherigen Ansprüche, wobei das Pflegemittel bzw. das Reinigungs- und/oder Desinfektionsmittel ein Mittel enthält, welches eine Vermischung des Pflegemittels mit einem Reinigungs- und/oder Desinfektionsmittel bzw. des Reinigungs- und/oder Desinfektionsmittels mit einem Pflegemittel gewährleistet,
**dadurch gekennzeichnet,**
daß das Mittel ein ethoxiliertes Olein, eine Kombination aus Fettaminpolyglykol und einem ethoxilierten Olein oder ein ethoxiliertes Polyalkylenglykolether ist.

9. Pflegemittel oder Reinigungs- und/oder Desinfektionsmittel nach Anspruch 8,
**dadurch gekennzeichnet,**
daß der Anteil des Mittels etwa 0,5% bis 15% beträgt.

10. Pflegemittel oder Reinigungs- und/oder Desinfektionsmittel nach Anspruch 9,
**dadurch gekennzeichnet,**
daß der Anteil des Mittels etwa 1% bis 10% beträgt.

11. Pflegemittel oder Reinigungs- und/oder Desinfektionsmittel nach Anspruch 10,
**dadurch gekennzeichnet,**
daß der Anteil des Mittels etwa 4% bis 8% beträgt.

12. Pflegemittel nach einem der Ansprüche 8 bis 11,
**dadurch gekennzeichnet,**
daß das Pflegemittel ein Pflege- oder Schmieröl ist.

13. Pflegemittel nach einem der Ansprüche 8 bis 11,
**dadurch gekennzeichnet,**
daß das Pflegemittel ein Spray ist.

## Claims

1. Process for cleaning and/or disinfecting and protecting an instrument for medical treatment, particularly a hollow or tubular instrument for medical treatment, in particular a medical or dental treatment instrument such as a handpiece, wherein the treatment instrument is cleaned and/or disinfected with a preferably liquid cleaning and/or disinfecting agent and is then protected with a protecting agent,
**characterised in that**
use is made of a cleaning agent and/or a disinfecting agent and/or a protecting agent, in any or all of which an agent is contained which guarantees intermixing of the cleaning and/or disinfecting agent with the protecting agent.

2. Process according to Claim 1,
**characterised in that**
use is made of a solvent or emulsifier by way of agent in the cleaning and/or protecting agent.

3. Process according to Claim 1 or 2,
**characterised in that**
use is made of an oil or a spray by way of protecting agent.

4. Process according to one of Claims 1 to 3,
**characterised in that**
use is made of a mixing ratio for the agent amounting to about 1 % to 10 %, in particular about 5 %, in the liquid and/or in the protecting agent.

5. Process according to one of the preceding claims,
**characterised in that**
use is made of an ethoxylated olein by way of agent.

6. Process according to one of the preceding claims,
**characterised in that**
the protecting agent is introduced or injected manually or in a controlled device for cleaning and/or disinfecting and protecting the treatment instrument.

7. Process according to one of the preceding claims,
**characterised in that**
it is implemented in a rinsing container, preferably in a rinsing container filled with the cleaning and/or disinfecting liquid.

8. Protecting agent or liquid cleaning and/or disinfecting agent for carrying out the process according to one of the preceding claims, wherein the protecting agent or alternatively the cleaning and/or disinfecting agent contains an agent which guarantees intermixing of the protecting agent with a cleaning and/or disinfecting agent or alternatively of the cleaning and/or disinfecting agent with a protecting agent,
**characterised in that**
the agent is an ethoxylated olein, a combination of fatty-amine polyglycol and an ethoxylated olein or an ethoxylated polyalkylene glycol ether.

9. Protecting agent or cleaning and/or disinfecting agent according to Claim 8,
**characterised in that**
the proportion of the agent amounts to about 0.5 % to 15 %.

10. Protecting agent or cleaning and/or disinfecting agent according to Claim 9,
**characterised in that**
the proportion of the agent amounts to about 1 % to 10 %.

11. Protecting agent or cleaning and/or disinfecting agent according to Claim 10,
**characterised in that**
the proportion of the agent amounts to about 4 % to 8 %.

12. Protecting agent according to one of Claims 8 to 11,
**characterised in that**
the protecting agent is a protecting or lubricating oil.

13. Protecting agent according to one of Claims 8 to 11,
**characterised in that**
the protecting agent is a spray.

## Revendications

1. Procédé de nettoyage et/ou de désinfection et d'entretien d'un instrument médical qui est en particulier creux ou tubulaire, en particulier d'un instrument médical utilisé en médecine ou en dentisterie tel qu'une pièce manuelle, dans lequel l'instrument est nettoyé et/ou désinfecté avec un produit de nettoyage et/ou de désinfection qui est de préférence liquide et est ensuite entretenu avec un produit d'entretien, lequel procédé est caractérisé en ce que l'on utilise un produit et nettoyage et/ou de désinfection et/ou un produit d'entretien qui contien(nen)t une substance qui assure le bon mélange du produit de nettoyage et/ou de désinfection avec le produit d'entretien.

2. Procédé selon la revendication 1, caractérisé en ce que l'on utilise comme substance contenue dans le produit de nettoyage et/ou d'entretien un solvant ou un agent émulsionnant.

3. Procédé selon la revendication 1 ou 2, caractérisé en ce que l'on utilise comme produit d'entretien une huile ou un spray.

4. Procédé selon l'une quelconque des revendications 1 à 3, caractérisé en ce que le rapport de mélange de la substance dans le liquide et/ou dans le produit d'entretien est compris entre environ 1 % et 10 % , et est en particulier égal à 5 %.

5. Procédé selon l'une quelconque des revendications précédentes, caractérisé en ce que l'on utilise comme substance une oléine éthoxylée.

6. Procédé selon l'une quelconque des revendications précédentes, caractérisé en ce que le produit d'entretien est introduit ou insufflé manuellement ou à l'aide d'un dispositif commandé de nettoyage et/ou de désinfection et d'entretien de l'instrument médical.

7. Procédé selon l'une quelconque des revendications précédentes, caractérisé en ce qu'il est réalisé dans un récipient de lavage/rinçage, de préférence dans un récipient de lavage/rinçage rempli avec le liquide de nettoyage et/ou de désinfection.

8. Produit d'entretien ou produit liquide de nettoyage et/ou de désinfection permettant de réaliser le procédé selon l'une des revendications précédentes, le produit d'entretien ou produit de nettoyage et/ou de désinfection contenant une substance qui assure le bon mélange du produit d'entretien avec un produit de nettoyage et/ou de désinfection, ou du produit de nettoyage et/ou de désinfection avec un produit d'entretien, caractérisé en ce que la substance est une oléine éthoxylée, une combinaison d'un polyglycol d'une amine grasse et d'une oléine éthoxylée ou un polyalkylèneglycoléther éthoxylé.

9. Produit d'entretien ou produit de nettoyage et/ou de désinfection selon la revendication 8, caractérisé en ce que la proportion de la substance est comprise entre environ 0,5 % et 15 %.

10. Produit d'entretien ou produit de nettoyage et/ou de désinfection selon la revendication 9, caractérisé en ce que la proportion de la substance est comprise entre environ 1 % et 10 %.

11. Produit d'entretien ou produit de nettoyage et/ou de désinfection selon la revendication 10, caractérisé en ce que la proportion de la substance est comprise entre environ 4 % et 8 %.

12. Produit d'entretien selon l'une des revendications 8 à 11, caractérisé en ce que le produit d'entretien est une huile lubrifiante ou une huile d'entretien.

13. Produit d'entretien selon l'une des revendications 8 à 11, caractérisé en ce que le produit d'entretien est un spray.
